**Europäisches Patentamt**

**European Patent Office**

**Office européen des brevets**

(11) Veröffentlichungsnummer: **0 250 513 B1**

# EUROPÄISCHE PATENTSCHRIFT

(45) Veröffentlichungstag der Patentschrift:
**11.09.91 Patentblatt 91/37**

(51) Int. Cl.⁵ : **C12N 15/00**

(21) Anmeldenummer: **87900133.7**

(22) Anmeldetag : **19.12.86**

(86) Internationale Anmeldenummer:
**PCT/EP86/00763**

(87) Internationale Veröffentlichungsnummer:
**WO 87/03904 02.07.87 Gazette 87/14**

(54) **VERFAHREN ZUR VERBESSERUNG DER EXPRESSION VON EUKARYONTENPROTEIN.**

Verbunden mit 86117750.9/0227064 (europäische Anmeldenummer/Veröffentlichungsnummer) durch Entscheidung vom 16.06.89.

(30) Priorität : **19.12.85 DE 3545126**

(43) Veröffentlichungstag der Anmeldung :
**07.01.88 Patentblatt 88/01**

(45) Bekanntmachung des Hinweises auf die Patenterteilung :
**11.09.91 Patentblatt 91/37**

(84) Benannte Vertragsstaaten :
**AT BE CH DE ES FR GB GR IT LI LU NL SE**

(56) Entgegenhaltungen :
**EP-A- 0 108 667**
**EP-A- 0 159 714**
**WO-A-86/05514**

(56) Entgegenhaltungen :
GENE, Band 33, Nr. 3, 4. April 1985, Elsevier Science Publishers (Amsterdam, NL); M.J. Browne et al.:"Isolation of a human tissue-type plasminogen-activator genomic DNA clone and its expression in mouse L cells", Seiten 279-284
GENE, Band 24, 1983, Elsevier Science Publishers (Amsterdam, NL); J.H. Lupker et al.:"Abundant excretion of human growth hormone by recombinant-plasmid-transformed monkey kidney cells", Seiten 281-287

(73) Patentinhaber : BOEHRINGER MANNHEIM GMBH
Patentabteilung, Abt. E Sandhofer Strasse 112-132 Postfach 31 01 20
W-6800 Mannheim 31 Waldhof (DE)

(72) Erfinder : SCHUMACHER, Günter
Kapellenweg 20
W-8139 Bernried (DE)
Erfinder : HIRTH, Peter
Winkstrasse 6
W-8000 München 70 (DE)
Erfinder : BUCKEL, Peter
Eichenstrasse 19
W-8131 Bernried (DE)

(74) Vertreter : Huber, Bernhard, Dipl.-Chem. et al
Patentanwälte H. Weickmann, Dr. K. Fincke F.A. Weickmann, B. Huber Dr. H. Liska, Dr. J. Prechtel Möhlstrasse 22 Postfach 860 820
W-8000 München 86 (DE)

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Verbesserung der Expression von Eukaryontenprotein, wobei man cDNA eines Eukaryontengens in Prokaryonten kloniert, die klonierte cDNA daraus gewinnt und gegebenenfalls dieselbe in eine Eukaryontenzelle einführt, welche die cDNA in das gewünschte Protein übersetzt.

Die Expression von Eukaryonten-DNA in tierischen oder menschlichen Zellkulturen kann von vielen Faktoren abhängen. Entscheidend hierfür können Promotor-Enhancer-Elemente, Terminationssequenzen und Polyadenylierungs-sequenzen und zellspezifische Faktoren sein. Diese zellulären Faktoren und DNA-Strukturen spielen eine Rolle in der Übersetzung von DNA in mRNA. Dabei ist es bei Eukaryontenzellen und damit auch beim Menschen mit wenigen Ausnahmen so, daß die RNA im Zellkern als Vorläufer-RNA synthetisiert wird. Diese Vorläufer-RNA enthält, wie die Ursprungs-DNA, Teile, die Informationen exprimieren und Teile, die keine Information tragen und als Introns bezeichnet werden. Die Vorläufer-RNA wandert durch den Kern und wird dann im Cytoplasma durch einen Vorgang, der als "Spleißen" bezeichnet wird, in eine meist kürzere Form durch Herausschneiden der Introns und Wiederverknüpfung der dabei gebildeten neuen Enden in eine mRNA überführt, die nun ein Protein mit katalytischen oder strukturellen Eigenschaften kodieren kann. Auch diese Übersetzung der mRNA in das Protein kann von Faktoren wie Messenger-Stabilität, Konformation der RNA oder Qualität der Nukleotide zur Initiation und gegebenenfalls auch der Termination der Proteinsynthese abhängig sein. Es ist nun sehr wünschenswert, die Expression beeinflussen zu können, um auf diesem Weg die Synthese der gewünschten Proteine steigern zu können.

Es war schon bekannt, in eine cDNA außerhalb der kodierenden Region und zwar am Ende ein Intron anzuhängen. Eine Wirkung auf die Expression des Genprodukts wurde dabei nicht festgestellt. Weiterhin tritt bei manchen Hefen ein Gen auf, das in einigen Stämmen ein Intron enthält und in anderen nicht. Es wurde deshalb versucht, das intronhaltige Gen aus einem Hefestamm in einen anderen Hefestamm einzuführen, der dieses entsprechende Gen ohne Intron enthält. Dabei wurde festgestellt, daß die Expression verschlechtert wird.

Es war daher ein Ziel der Erfindung ein Verfahren zu finden, mit dessen Hilfe die Expression von Eukaryontenprotein beeinflußt werden kann, so daß auf diesem Wege die Proteinsynthese verbessert wird.

Dieses Ziel wird erreicht durch ein Verfahren zur gegenüber cDNA verbesserten Expression von Eukaryontenprotein, wobei man cDNA eines Eukaryontengens in Prokaryonten kloniert, die klonierte cDNA daraus gewinnt und dieselbe in eine Eukaryontenzelle einführt, welche die cDNA in das gewünschte Protein übersetzt, das dadurch gekennzeichnet ist, daß man mindestens ein im entsprechenden Gen natürlich vorkommendes Intron gewinnt, in die kodierende Sequenz an geeigneter Stelle der cDNA einligiert und die so erhaltene Intron-haltige cDNA in Prokaryonten kloniert.

Überraschenderweise zeigt sich, daß nicht nur die bekannten Faktoren die Genexpression beeinflussen können, sondern daß es vielmehr möglich ist, mit der Einführung eines Introns in eine kodierende cDNA eine Steigerung der Expression und damit der Proteinsynthese zu bewirken.

Zur Durchführung des erfindungsgemäßen Verfahrens stellt man die das gewünschte Protein kodierende cDNA in an sich bekannter Weise her z. B. unter Verwendung der entsprechenden mRNA. Diese cDNA, die ein zu synthetisierendes Protein kodiert, wird in an sich bekannter Weise in einem Prokaryonten kloniert. Dazu wird die cDNA mit Hilfe eines Plasmidvektors in den Prokaryonten eingeführt. In die das gewünschte Protein kodierende cDNA, die eine Kopie der entsprechenden mRNA ist, wird jedoch vor Einführung in die Prokaryontenzelle ein Intron insertiert, das in dem das gewünschte Protein kodierenden Gen der Eukaryontenzelle natürlich vorkommt. Das oder die Introns werden z. B. aus einer Genbank einer eukaryontischen Zelle über Hybridisierung mit einem Teil der cDNA gewonnen. Der Vektor, der die cDNA einschließlich des Introns trägt, wird in dem Prokaryonten kloniert. Die klonierte DNA wird dann gewonnen und kann gegebenenfalls in eine Eukaryontenzelle eingeführt werden. In der Eukaryontenzelle exprimiert dann die von der DNA abgeleitete mRNA das gewünschte Protein.

Zur Durchführung des erfindungsgemäßen Verfahrens ist es auch möglich, in die das gewünschte Protein kodierende cDNA mehrere Introns einzuführen, die in der das gewünschte Protein kodierenden natürlich vorkommenden DNA enthalten sind.

Bevorzugt ist es, ein oder mehrere Introns des das Protein kodierenden Gens in die entsprechende cDNA zu insertieren, so daß das oder die Introns sich in der cDNA an derselben Stelle befinden, an der sie auch in dem natürlichen Gen liegen.

In einer bevorzugten Ausführungsform wird das erfindungsgemäße Verfahren durchgeführt unter Verwendung einer cDNA, die die Proteinase tPA kodiert. Dazu wird entweder ein Plasmid verwendet, das das tPA-Gen enthält oder die tPA kodierende cDNA wird in bekannter Weise über mRNA gewonnen. Ein oder mehrere Introns, die das tPA-Gen in natürlichen Eukaryontenzellen enthält, werden in diese tPA-cDNA einligiert. Die Plasmide, die die tPA-cDNA mit einem oder mehreren Introns enthalten, stellen die für die Expression in Eukaryonten benutzten Shuttle-Vektoren dar. Diese Shuttle-Vektoren werden in Eukaryontenzellen transfektiert und exprimieren dann tPA. Überraschenderweise ist die Expression von tPA in Eukaryontenzellen, die tPA kodierende Plasmide mit Introns enthalten, bei weitem höher als die vergleichbarer Eukaryontenzellen mit tPA kodierenden Plasmiden ohne Introns.

Bevorzugt wird in die das Protein tPA kodierende cDNA das Intron C einligiert. Die Sequenz der tPA-cDNA ist bekannt und wurde von Pennica et al (Nature 1983, 301, 214-221) publiziert. In dieser publizierten Sequenz liegt ein Intron, nämlich das von Ny et al (Proc. Natl. Acad. Sci., 81, 5355, 1984) beschriebene Intron C zwischen der in der cDNA angegebenen Bgl II Schnittstelle an Pos. 187 und der Pst I Schnittstelle an Pos. 204. Aus dieser Beschreibung ergibt sich, daß Intron C an beiden Seiten von Exon-Sequenzen umgeben ist, nämlich zur 5'-Richtung hin von Exon-Sequenzen, die eine Bgl II-Spaltstelle kodieren und zum 3'-Ende hin von Exon-Sequenzen, die eine Pst I-Schnittstelle enthalten. In einer bevorzugten Ausführungsform des erfindungsgemäßen Verfahrens wird das Intron C über die auch im Gen enthaltenen Bgl II- und Pst I-Schnittstellen in die cDNA einligiert in an sich bekannter Weise.

Um die Intronsequenz für tPA zu erhalten, wird bevorzugt in bekannter Weise über mRNA eine cDNA-Sequenz hergestellt, die den als tPA bezeichneten Tissue Plasminogen Activator codiert. Durch Spaltung mit einer Restriktionsendonuklease wird aus dieser tPA-cDNA ein beliebiges Fragment gewonnen, oder ein solches, das durch die Pst I Schnittstelle an Pos. 204 und Pos. 618 der von Pennica et al. publizierten Sequenz charakterisiert ist. Dieses Fragment wird in einen Plasmidvektor ligiert. Der Plasmidvektor wird dann in einen Wirtsstamm transformiert und dann in bekannter Weise selektioniert, beispielsweise über Resistenzgene. Ein derartig selektierter Wirtsstamm, der das Plasmid mit dem tPA-cDNA-Fragment enthält, wird dann mit einem Lambda-Phagenlysat infiziert (R.M. Lawn et al, Cell 15 (1978) 1157)). Es wird dabei ein Lambda-Phagenlysat verwendet, in dessen DNA vorher genomische DNA aus menschlichen Zellen einligiert wurde. Die Vorgehensweise erfolgt exakt nach der von Maniatis, T., Fritsch, E.F., und Sambrook, J. in: "Molecular Cloning", Cold Spring Harbor Laboratory 1982, S. 353-361 beschriebenen Methode. Die nach dem in vivo Rekombinationsereignis enthaltenen Phagen können nur durch Oligonukleotide, die von der tPA-cDNA abgeleitet wurden, charakterisiert werden. Das im vorliegenden Fall isolierte Teilfragment des tPA-Gens wird mit Eco RI gespalten und das resultierende 9 kb große Fragment in den ebenfalls mit Eco RI gespaltenen Plasmidvektor pBR 322 ligiert. Das resultierende Plasmid trägt die Bezeichnung pBT 72, = DSM 3609.

Aus diesem Plasmid kann dann durch Restriktionsendonukleasen wie z. B. Bgl II und Pst I der ca. 2,0 kb große dem Intron C entsprechende Teil des tPA-Gens inklusive der angrenzenden Exonstrukturen herausgeschnitten werden und für die weitere Klonierung verwendet werden. Dieses so gewonnene Intron C-Fragment wird dann in eine in bekannter Weise gewonnene cDNA, die tPA kodiert, eingefügt. Bevorzugt wird dieses Intron an seiner im Gen vorkommenden Position inseriert. Dazu wird die tPA-cDNA an der entsprechenden Stelle durch dieselben Restriktionsendonukleasen wie das Intron C mit seinen angrenzenden Exonstrukturen gespalten, also z. B. Bgl II an Pos. 187 und Pst I an Pos. 204 und das in oben beschriebener Weise gewonnene Intron C dann eingefügt. Alternativ kann, falls die Sequenz des einzufügenden Introns bekannt ist, letzteres auch synthetisch hergestellt und in das Verfahren eingesetzt werden.

Zur Gewinnung einer tPA-Sequenz mit Intron wird bevorzugt das Plasmid pBT 95 (DSM 3611) verwendet. Dieses Plasmid besteht aus einem Plasmidvektor und trägt die komplette cDNA-Sequenz, die zur Kodierung von tPA notwendig ist einschließlich des Signalpeptids.

In dieses Plasmid pBT 95 wird das Intron C in an sich bekannter Weise ligiert. Ein Plasmid, das sowohl die tPA kodierende Sequenz als auch das Intron C enthält, ist das erfindungsgemäße Plasmid pBT 94 (DSM 3610).

Aus dem Plasmid pBT 94 kann durch Spaltung mit Restriktionsendonukleasen wie z. B. Xba I und Hind III die tPA kodierende Sequenz mit Intron leicht in an sich bekannter Weise herausgespalten und umkloniert werden. Sofern das Intron eine der beiden Restriktionserkennungsstellen erkennt, kann das Plasmid unter limitierenden Bedingungen gespalten werden oder die Erkennungsstelle im Intron vorher zerstört werden, z. B. durch Auffüllen der überstehenden Enden mit allen vier Triphosphaten in Gegenwart von DNA-Polymerase.

Zur Expression von tPA in Eukaryontenzellen wird ein Plasmid-Shuttle-Vektor verwendet. In diesen Shuttle-Vektor, der alle Funktionen trägt, um sowohl in Prokaryontenzellen als auch in geeigneten Eukaryontenzellen vermehrt zu werden, wird bevorzugt das aus pBT 94 erhaltene Fragment, das die gesamte tPA kodierende Sequenz und das Intron C trägt, einligiert. Die erhaltenen Plasmide werden zur Transfektion von Eukaryontenzellen verwendet. Besonders bevorzugt wird als Shuttle-vektor das erfindungsgemäße Plasmid pBT 102 (DSM 3612) verwendet, das sowohl die gesamte tPA kodierende Sequenz mit dem Intron C als auch die für die Expression in Eukaryontenzellen notwendigen Gene enthält. Neben diesen für die transiente Expression geeigneten Vektoren können auch Vektoren verwendet werden, die für die stabile Integration in Eukaryonten DNA geeignet sind.

Die Transfektion der Eukaryontenzellen erfolgt in an sich bekannter Weise. Mit den erfindungsgemäß verwendeten Vektoren, die die gesamte tPA kodierende Sequenz einschließlich des Introns C tragen, werden ausgezeichnete Ergebnisse bei der Expression erhalten. Zum Vergleich wurde in denselben Shuttle-Vektor das Plasmid pBT 95, das die gesamte tPA kodierende Sequenz ohne Intron enthält, ligiert. Auch dieses Plasmid wurde in Eukaryontenzellen transfektiert. Dabei ergaben sich für die Expression weitaus schlechtere Ergebnisse.

Mit dem erfindungsgemäßen Verfahren ist es möglich, die Expression von Eukaryontenprotein bedeutend zu verbessern. Weiterhin werden erfindungsgemäß Plasmide zur Verfügung gestellt, die zur Herstellung von tPA mit hohen Ausbeuten geeignet sind.

Die Erfindung wird durch die Beispiele in Verbindung mit der Zeichnung erläutert. In dieser zeigen:

Fig. 1 das Plasmid pBT 95, welches die komplette cDNA, ligiert in pHC 79, enthält,

Fig. 2 das Plasmid pBT 94, welches, zusätzlich zu den Sequenzen aus pBT 95, das Intron C enthält,

Fig. 3 das Plasmid pBT 102, welches für die Expression in Eukaryonten geeignet ist und die tPA-cDNA sowie Intron C enthält,

Fig. 4 das Plasmid pBT 103; analog pBT 102, jedoch ohne Intron,

Fig. 5 das Plasmid pKCR-$\overrightarrow{tPA}_1$,

Fig. 6 das Plasmid pKCR-$\overrightarrow{tPA}_2$.

Fig. 7 das Plasmid pSVßG und seine Herstellung

Fig. 8 das Plasmid pSVßc und seine Herstellung

Fig. 9 das Plasmid RßG ref

Fig. 10 S1-mapping mit einer aus Plasmid pßcg gewonnenen Probe

Die Fragmentgrößen in den Plasmiden sind nicht maßstabgerecht dargestellt. Auußerdem sind nur die für die Klonierung relevanten Erkennungssequenzen angegeben.

## Beispiel 1

Isolierung eines tPA Gen-Fragments

Nach der von Maniatis, T., Fritsch, E.F., und Sambrook, J. in: "Molecular Cloning", Cold Spring Harbor Laboratory 1982, S. 353-361 beschriebenen Methode wird über ein in vivo Rekombinationsereignis aus der Maniatis Genbank (R.M. Lawn, E.F. Fritsch, R.C. Parker, G. Blake and T. Maniatis, Cell 15 (1978), 1157) ein Lambda-Phagen gewonnen, der einen Teil des tPA-Gens kodiert. Dazu wird ein DNA-Fragment der tPA-cDNA, vorzugsweise das ca. 400 Bp große durch die Pst I Schnittstelle an Pos. 204 und Pos. 618 charakterisierte Fragment isoliert und in den von B. Seed (Nuc. Acid Res., 11, 2427, 1983) beschriebenen und ebenfalls mit Pst I gespaltenen Vektor Pi AN 7 ligiert. Dieser einen Teil der tPA-cDNA enthaltende Vektor wird in einem E. coli-Stamm (W 3110, P3) transformiert, in dem auf Erhalt dieses Plasmids selektioniert werden kann. Nach Überinfektion mit λ-Phagen, in die zuvor Teile des menschlichen Genoms einligiert wurden (R.M. Lawn, E.F. Fritsch, R.C. Parker, G. Blake and T. Maniatis, Cell 15 (1978), 1157), kann es zu einem Rekombinationsereignis zwischen einem Teil des tPA-cDNA enthaltenden Plasmids und einer λ-DNA kommen. Bevorzugt findet ein solches Rekombinationsereignis über homologe Sequenzen, z. B. tPA-Sequenzen, statt. Im vorliegenden Fall konnten aus der Maniatis Genbank (R.M. Lawn, E.F. Fritsch, R.C. Parker, G. Blake and T. Maniatis, Cell 15 (1978), 1157) Phagen isoliert werden, die einen Teil des tPA-Gens enthalten. Das Prinzip der Klonierung eines Genfragments über ein in vivo Rekombinationsereignis ist in Maniatis, T., Fritsch, E.F., und Sambrook, J. in: "Molecular Cloning", Cold Spring Harbor Laboratory 1982, S. 353-361 beschrieben. Die so erhaltene λ-DNA wird mit Eco RI gespalten. Durch Hybridisierung mit Oligonukleotiden, die der Pos. 390-410 bzw. 609-627 der von Pennica et al. publizierten Sequenz (Nature 1983, 301, 214-221) entsprechen, kann ein 9 kb großes Eco RI-Fragment charakterisiert und in das mit Eco RI gespaltene Plasmid pBR 322 kloniert werden; dabei erhält man pBT 72, DSM 3609. Aus diesem Plasmid kann durch Spalten mit Bgl II und Pst I ein ca. 2 kb großes Fragment erhalten werden, das aus dem Intron C und zusätzlich aus den angrenzenden Exonsequenven besteht, die eine Bgl II und Pst I Erkennungssequenz besitzen.

## Beispiel 2

Klonierung von tPA-kodierenden Sequenzen mit Intron

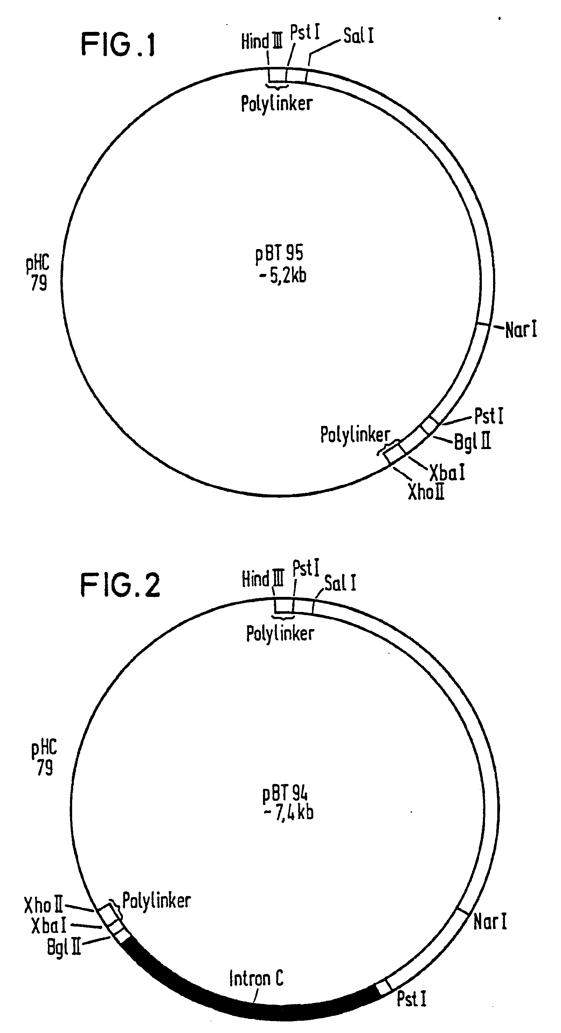

Das Plasmid pBT 95, DSM 3611 (Fig. 1) wird hergestellt, indem die gesamte tPA-cDNA in das Plasmid pHC 79 ligiert wird. Aus Gründen der Umklonierbarkeit wurden an die Enden kommerziell erhältliche Polylinkerregionen aus pUC 12 ligiert. Ebenso können andere Polylinker verwendet werden, die Restriktionserkennungssequenzen enthalten, welche weder in Intron noch in der cDNA vorkommen.

Das Plasmid pBT 95 wird mit Pst I teilweise und mit Bgl II vollständig gespalten. Das nach Beispiel 1 erhaltene, ca. 2 kb große Fragment, welches das Intron C vollständig enthält, wird in das gespaltene Plasmid pBT 95 ligiert. Das resultierende Plasmid ist pBT 94 (DSM 3610), (Fig. 2).

**Beispiel 3**

Konstruktion von tPA kodierenden Plasmiden zur Expression in Eukaryontenzellen:

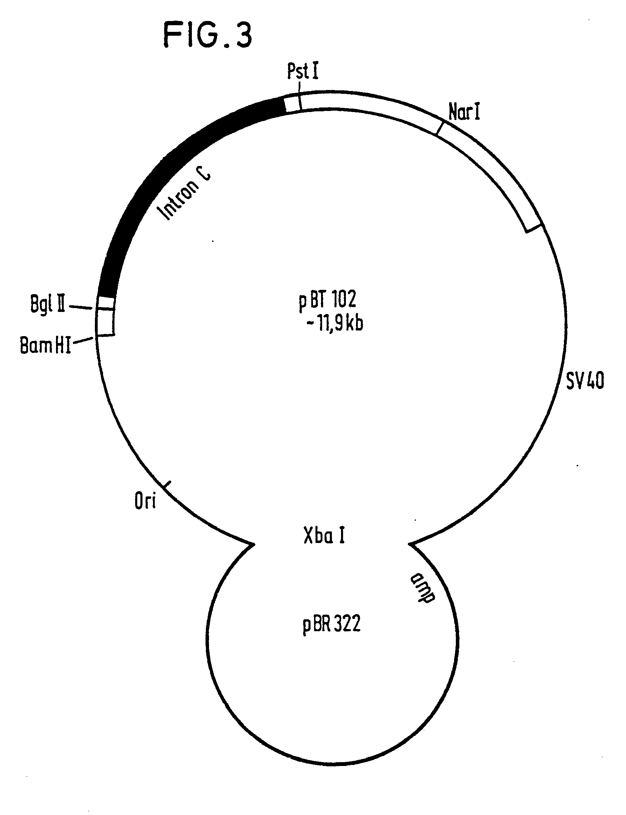

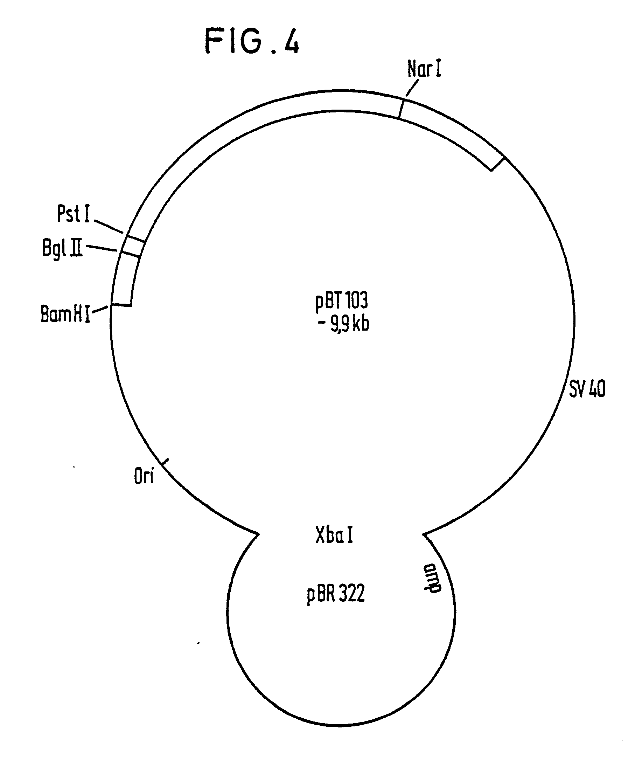

Zur Expression in Eukaryontenzellen wird Plasmid pA-11-SVL$_2$ (DSM 3614) verwendet (Gruß et al, in: "Eukaryotic Viral Vectors" Gluzman herausgeber, S. 13-18, Cold Spring Harbor Laboratory, 1982). Dieses Plasmid trägt im wesentlichen Sequenzen des SV40-Virus, die späten Funktionen zwischen der Bam HI-Schnittstelle an Position 282 und 2469 wurden deletiert. Ein mit Eco RI gespaltener und mit Xba I- Linkern versehener Vektor pBR 322 wurde in die Taq- Schnittstelle von pA-11-SVL$_2$ an Position 4675 ligiert. Der erhaltene Vektor trägt somit alle Funktionen um sowohl in E. coli-Zellen als auch in geeigneten Eukaryontenzellen, wie z. B. CV-1 verwendet zu werden. Plasmid pA-11-SVL$_2$ wird mit Bam HI gespalten, die Enden mit DNA-Polymerase und den 4 Triphosphaten glatt gemacht. In diese nun glatten Enden werden die ebenfalls auf gleiche Weise glatt gemachten Enden der mit Hind III- und Xba I-gespaltenen Fragmente aus pBT94 bzw. pBT95, die die für tPA kodierende DNA enthalten, wobei pBT 94 zusätzlich noch in die tPA kodierende Sequenz das Intron C einligiert hat, ligiert. Die resultierenden Plasmide tragen die Bezeichnung pBT 102 (DSM 3612), Fig. 3 mit Intron C bzw. pBT 103 (DSM 3613) (Fig. 4). Diese beiden Vektoren werden zum Vergleich der tPA-Expression in CV-1-Zellen verwendet. Vor der Transformation werden pBT 102 bzw. pBT 103 mit Xba I gespalten und in Konzentrationen von ca. 1 µg/l religiert. Durch diese Manipulation wird die Region, die das große T-Antigen des SV40 kodiert, wieder hergestellt. Die religierten Plasmide können zur Transfektion von CV-1-Zellen verwendet werden, da sie das große T-Antigen kodieren.

Dieses T-Antigen ist für die extrachromosomale Replikation dieser Vektoren essentiell. Zur Transfektion werden 4x10$^4$ CVI-Zellen auf kleinen Petrischalen ausgebracht. Am folgenden Tag werden die Zellen vor der Transfektion mit serumfreiem Medium (z. B. D-HEM-Medium) gewaschen. Die religierten Plasmide werden zusammen mit DEAE-Dextran auf die Zellen gegeben und 3 Stunden bei 37°C inkubiert (Sompayrac, L.M. and Danna, K.J. 1981 Proc. Natl. Acad. Sci U.S.A. 78, 7575-7578). Nach Waschen und Zugabe von frischem Medium kann nach 40stündiger Inkubation der Gehalt an tPA im Kultur-Überstand im ELISA- Test bestimmt werden. Die Anzahl der transfizierten und tPA-produzierenden Zellen wird mittels Immunstaining bestimmt. Dazu werden die entsprechenden Petrischalen mit PBS (phosphate buffered saline) gewaschen und nach Fixierung mit Formaldehyd mit 0,2 % Triton X 100 lysiert. Darauf wird mit Peroxidase gekoppelten, gegen tPA gerichteten Ziegen-IgG, inkubiert. Die gebundenen Antikörper werden dann durch die Peroxidase-spezifische Färbereaktion visualisiert. Im Mikroskop wird die Anzahl der tPA-produzierenden Zellen bestimmt.

Tabelle I zeigt die tPA-Expression im transienten System durch Plasmide, die nur die cDNA enthalten (pBT 103), bzw. die cDNA mit einem Intron enthalten (pBT 102).

## T a b e l l e  1

|         | Experiment 1 | Experiment 2 |
|---------|--------------|--------------|
| Ko      | -            | -            |
| pBT 102 | 2,4          | 2,92         |
| pBT 103 | 1,32         | 1,545        |

Die Einheiten sind angegeben in Adsorptionseinheiten bei 405 nm. Kontrolle: Keine Transfektion mit dem Vektor.

Den Daten der Tabelle ist zu entnehmen, daß mit Plasmid pBT 102 nahezu die doppelte Expressionsrate erhalten wird.

## Beispiel 4

Konstruktion der Expressions-Vektoren

Das Plasmid pKCR[1] (Herstellung vgl. Beispiel 5), welches den frühen SV40 - Promotor, daran anschließend das Kaninchen-ß-Globin-Gen und danach 2 Polyadenylierungsstellen enthält, wurde zwischen dem SV40-Promotor und dem ß-Globin-Gen mit Bam H 1 geschnitten, die Enden mit alkalischer Phosphatase aus Kälberdünn-Darm dephosphorysiert und das lineare Molekül durch zweimaliges Aufreinigen in Low melting Agarose isoliert. Die tPA c-DNA und das Mini-Gen wurden mit Xba und Hind III aus den rekonstituierten Plasmiden pBT 94 bzw. pBT 95 herausgeschnitten, die Enden mit dem Klenow-Fragment von DNA Polymerase und vier Deoxynucleosid-triphosphaten eingefüllt und anschließend auf Low melting Agarose gereinigt. Äquimolare Mengen von t-PA c-DNA und t-PA Mini-Gen wurden mit dem oben beschriebenen Vektor-Fragment mit T4 Ligase inkubiert und anschließend in E.coli HB101 (DSM 1607) transfektiert. DNA von Miniplasmid-Präparationen wurde durch Restriktions-Analyse auf t-PA Insertionen überprüft.

Es wurden Plasmide mit cDNA, bzw. Minigen in syn, als auch in anti-Orientierung zum frühen Promotor von SV40 isoliert.

Plasmid pKCR-tPA$_1$: tPAcDNA in syn Orientierung zum
frühen Promotor von SV40

Plasmid pKCR-tPA$_1$: tPAcDNA in anti Orientierung zum
frühen Promotor von SV40

Plasmid pKCR-tPA$_2$: tPA Minigen in syn Orientierung zum
frühen Promotor von SV40

Plasmid pKCR-tPA$_2$: tPA Minigen in anti Orientierung zum
frühen Promotor von SV40

Transkription nach Einführen der Expressions-Plasmide in Säuger-Zellen

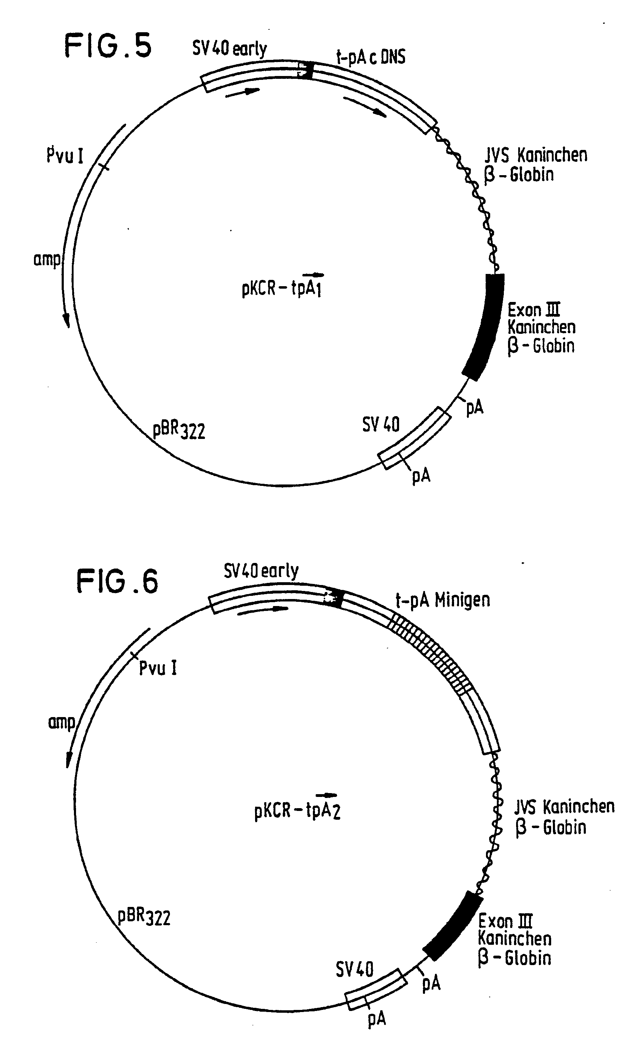

tPA cDNA und tPA Mini-Gen enthalten jeweils am 5'-Ende sieben Nucleotide untranslatierte Region. Sowohl cDNA als auch Mini-Gen haben keine Polyadenylierungs-Stelle. Im Falle des tPA cDNA Expressions-Vektors starten die Transkripte auf dem frühen Promotor von SV40, laufen in die tPA cDNA, dann wird das große Intron von kaninchen-ß-Globin herausgespleißt, schließlich terminieren sie an einer der beiden Polyadenylierungsstellen (siehe Fig. 5). Im Falle des tPA Minigen Expressions-Vektors werden die Transkripte zusätlich in der tPA Region gespleißt (siehe Fig. 6).

Expression von tPA nach Einführen der Expression-Plasmide in COS-Zellen.

COS-Zellen sind Affen-Zellen, die konstitutiv das große T-Antigen von SV40 synthetisieren (2). Plasmide, welche einen Replikations-Ursprung von SV40 besitzen, vermehren sich in diesen Zellen (2). Die oben beschriebenen Expressions-Plasmide wurden mit der DEAE-Dextran Methode (3) in COS-Zellen eingeführt und die tPA Konzentration in den Kultur-Überständen sechs Tage nach der Konzentration durch ELISA = Enzyme Linked Immunoadsorbant Assay bestimmt.

Die Transfektionen mit den einzelnen Expressions-Plasmiden wurden jeweils acht-fach durchgeführt. Es zeigte sich eine sehr gute Reproduzierbarkeit der einzelnen Transfektionen.

| Expressions-Vektor | Absorption bei 405 nm |
|---|---|
| pKCR-tPA₁ (→) | 0.7 - 0.75 |
| pKCR-tPA₁ (←) | 0.05 |
| pKCR-tPA₂ (→) | 1.2 - 1.25 |
| pKCR-tPA₂ (←) | 0.05 |

Bei diesen Experimenten wurden jeweils $10^5$ Zellen mit 2 µg DNA transfektiert.

Die Experimente zeigen, daß durch Einführung eines Introns in die tPA cDNA gesteigerte Expression von tPA auftritt.

(1) O'Hare, K., Benoist, C., Breathnach, R., 1981
Proc. Natl. Acad. Sci. USA 78, 1527 - 1531
(2) Gluzman, Y., 1981
Cell 23, 175 - 182
(3) Lai, C. Y., Nathans, D., 1974
Virology 60, 466 - 475

**Beispiel 5**

Der Einfluß von Introns auf die Expression von Kaninchen-ß Globinmessenger RNS in Säugerzellen.

In diesem Beispiel wird die Konstruktion von eukaryotischen Expressions-Vektoren für Kaninchen ß-Globin c-DNS und die genomische Sequenz von Kaninchen ß-Globin beschrieben. Die Expression der erwähnten Sequenzen erfolgte unter der Kontrolle des Simian Virus 40 frühen Promotors. Die Expressions-Plasmide wurden zusammen mit einem Referenz-Plasmid in Affennieren-Zellen transfektiert und die Globin-Transkripte durch S1-mapping bestimmt. Bei Vergleich von genomischem Expressions-Plasmid und c-DNS Expressions-Plasmid zeigt sich ein etwa 10facher Unterschied in der Menge der Globin Transkripte.

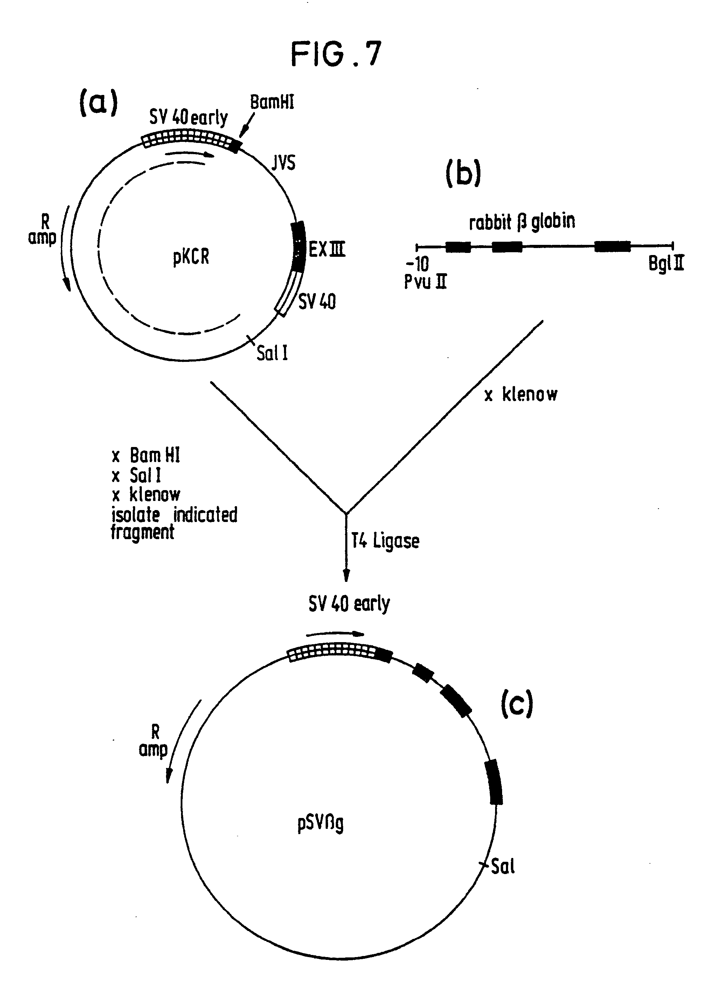

1) Konstruktion von Plasmid pSVßG (Fig.7)

a) Ausgangsplasmide:

Plasmid Z-pcRI/RchrßG-1 ist ein Plasmid, dessen Sequenz und Restriktionsschnittstellen in Science 206 (1979), 337-344 beschrieben sind.

pkCR ist ein Plasmid, dessen Konstruktion in Proc. Natl. Acad. Sci. 78 (1981), 1527-1531 beschrieben ist und das aus den dort beschriebenen, sequenzierten Elementen von pBR 322, SV 40 und dem Kaninchen ß-Globin Gen besteht.

b) Konstruktion:

Aus z-pcRI/RchrßG-1 wurde das Stück des ß-Globin-Gens, welches von der Pvu II(-10) Schnittstelle bis zur Bgl II Schnittstelle in der 3'-untranslatierten Region reicht, hergestellt, die Enden mit Klenow-Enzym eingefüllt und das Fragment auf einem Low-melting-Agarose-Gel über Vergleich mit den erwarteten Restriktionsenzymspaltmustern isoliert (Fig. 7b).

pkCR wurde mit Bam H I und Sal I geschnitten, die Enden mit Klenow-Enzym eingefüllt und das in Figur 7a gekennzeichnete Fragment auf einem Low-melting-Agarose-Gel nach Identifizierung über Restriktionsenzymspaltmuster identifiziert und isoliert.

Die beiden so erhaltenen Fragmente wurden mit T4-Ligase behandelt und in E.coli HB 101 (DSM 1607) transfektiert. Aus den erhaltenen Kolonien wird das Plasmid pSVßG über Restriktionsenzymspaltmuster identifiziert und isoliert (Fig. 7c)

In diesem Expressions-Plasmid ist die genomische Sequenz des Kaninchen ß-Globin-Gens (beginnend mit Position -10) mit dem frühen Promotor von Simianvirus SV 40 fusioniert.

Die Promotor-Elemente des Kaninchen ß-Globin-Gens sind bei dieser Fusion nicht mehr vorhanden. Die Termination der Transkripte erfolgt durch die Polyadenylierungs-Stelle des Kaninchen ß-Globin-Gens.

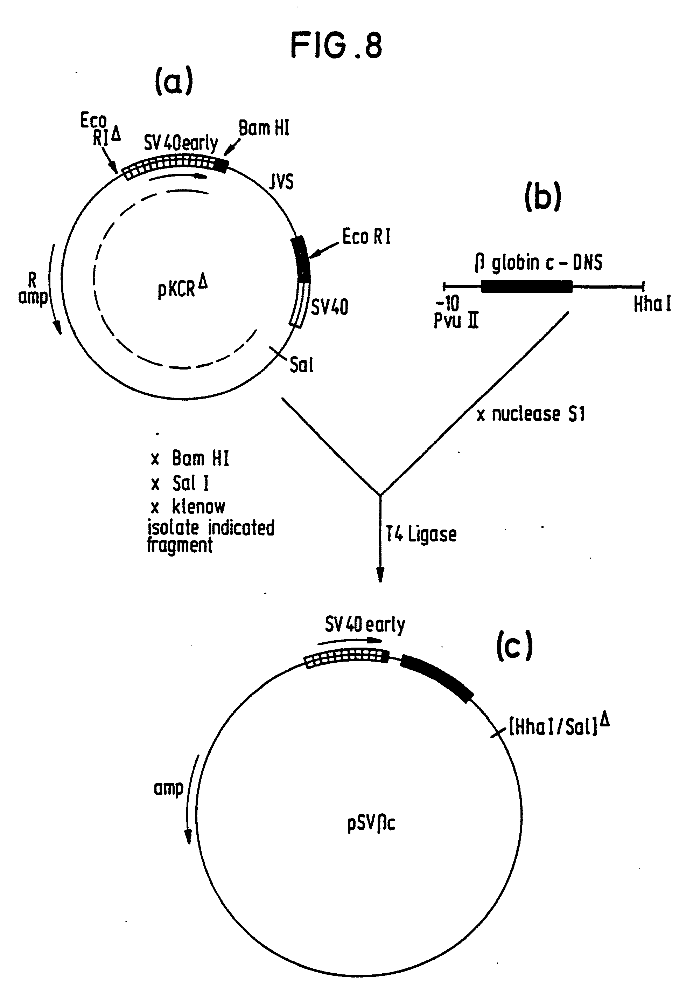

2) Konstruktion von Plasmid pSVßc (Fig. 8)

a) Ausgangsplasmide:

pkCR (vgl. Beispiel 5a)

pßcg: Plasmid, welches die Kaninchen ß-Globin c-DNS und zusätzliche Sequenzen bis maximal zur Position -100 der 5'-untranslatierten Region des Kaninchen ß-Globin-Gens enthält. Es ist also ein Hybrid aus genomischen Sequenzen der untranslatierten Region und der c-DNS des Kaninchen ß-Globin-Gens. Die Konstruktion erfolgt in der Weise, daß die vollständige ß-Globin c-DNS Sequenz, vgl. Cell 10, 571-585 (1977), und ein Teil der 5'-genomischen Sequenz des ß-Globin-Gens (Sequenz vgl. Science 206 (1979), 337 - 344) bis mindestens über die Pvu II(-10) Schnittstelle, aber maximal bis zur Position -100 ligiert wird.

b) Konstruktion

pkCR wurde mit Eco RI partiell gespalten, die Enden mit Nuclease SI behandelt, mit $T_4$-Ligase religiert, in E.coli HB 101 transfektiert und das Plasmid pkCR Δ über Restriktionsenzymspaltmuster identifiziert und isoliert (Fig. 8a)

pkCRΔ wurde mit Bam H I und Sal I geschnitten, die Enden mit Klenow-Enzym eingefüllt und das größere der beiden Fragmente auf einem Low-melting-Agarose-Gel isoliert. Aus Plasmid pßcg wurde das ß-Globin c-DNS-Fragment von Pvu II(-10) bis Hha I (Cell 10, 571-585) isoliert. Dieses Fragment enthält die gesamte 3' untranslatierte Region des Kaninchen ß-Globin Gens. Diese beiden Fragmente wurden mit $T_4$-Ligase behandelt und in E.coli HB 101 transfektiert. Aus den erhaltenen Kolonien wurde Plasmid pSVßc über Restriktionsenzymspaltmuster identifiziert und isoliert.

In Plasmid pSVßc ist also ein genomisch c-DNS Kaninchen ß-Globin Hybrid mit dem frühen Promotor von SV 40 an der Position -10 von Kaninchen ß-Globin fusioniert. Das Expressions-Plasmid enthält keine Introns. Es wird die Polyadenylierungsstelle von Kaninchen ß-Globin verwendet.

3) Vergleich von pSVßg und pSVßc

Die Fusion der ß-Globin Sequenzen mit dem frühen Promotor von Simian Virus 40 erfolgt in beiden Fällen an Position -10 von Kaninchen ß-Globin (+1 bezeichnet den Start der Transkripte). In beiden Fällen wird für die Termination die Polyadenylierungsstelle des Kaninchen ß-Globin Gens verwendet. pSVßg enthält die beiden Introns des Kaninchen ß-Globin Gens, pSVßc enthält keine Introns.

4) Transfektion von pSVßg und pSVßc in Säugerzellen

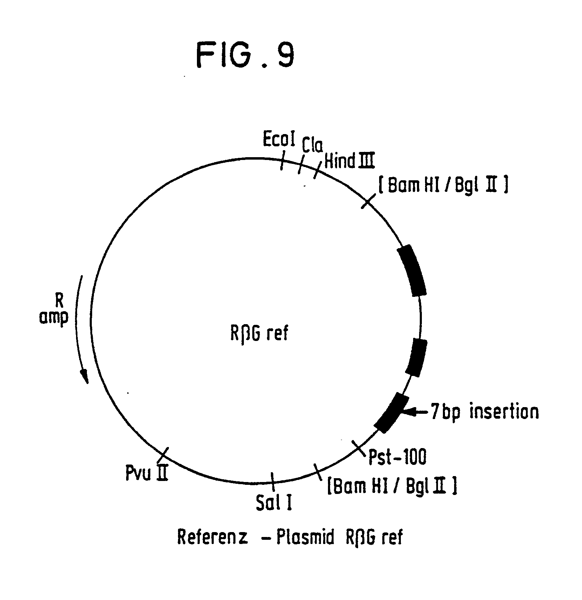

Als Referenz-Plasmid wurde das in Fig.9 dargestellte Plasmid verwendet. Die Bezeichnung ist RßGref. Es enthält das Kaninchen ß-Globin Gen in die Bam HI Stelle von $pBR_{322}$ inseriert. In Exon I befindet sich eine Insertion von sieben Basen-Paaren. Es wurden 10 µg pSVßg und 10 µg RßG ref gemischt und parallel dazu 10 µg pSVßc und 10 µg RßG ref und nach der Kalzium-phosphat Präzipitations-Methode (Virulogy 52 (1973), 456-467) in jeweils $2 \times 10^6$ Affennieren-Zellen (CV1) transfektiert. Die Zellen wurden 50 Stunden nach der Transfektion geerntet und RNS nach Standardmethode (Biochemistry 24 (1979) 5294-5299) isoliert.

5) S1-mapping

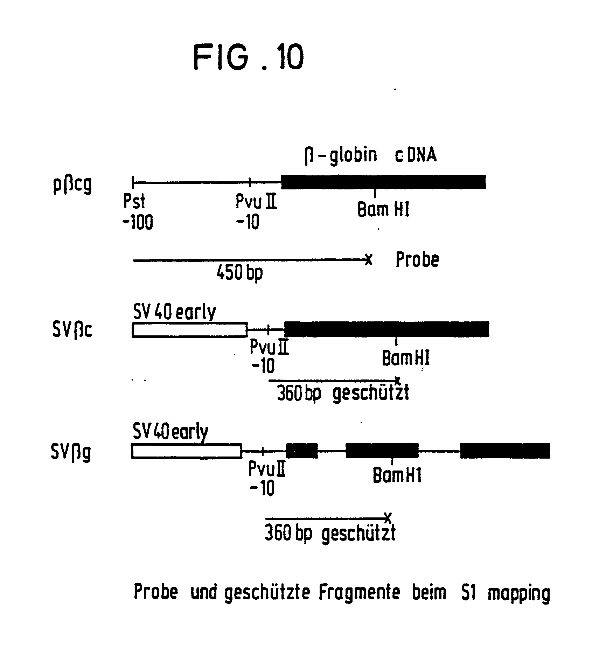

Das S1-mapping wurde nach Standard-Methode durchgeführt (Nucl. Acids Res. 7 (1979) 1175-1193) Präparation der Probe: pßcg (vgl. Fig. 10) ist ein Plasmid, welches einen Hybrid aus Sequenzen der untranslatierten Region des Kaninchen ß-Globin Gens bis zur Pst-Stelle bei -100 und die gesamte Kaninchen ß-Globin c-DNS enthält. pßcg wurde mit Bam HI geschnitten und anschließend mit alkalischer Phosphatase aus Kälber-Dünndarm dephosphatiert. Die Enden wurden mit Polynukleotid-Kinase und $\gamma^{32}P$ ATP kinasiert, mit Pst I nachgeschnitten und das interessierende 450 bp Bam HI-Pst Fragment auf einem Low-melting Agarose Gel isoliert. 50 000 cpm der endmarkierten Probe wurden mit jeweils 20 µg RNS hybridisiert (Bedingungen in 5 beschrieben). Die Hybride wurden mit 30 U S1 Nuklease behandelt und anschließend auf einem 4 % Polyacrylamid-Gel mit 7 M Harnstoff aufgetrennt und das Gel mit Röntgen-Film exponiert. Die Probe und die erwarteten geschützten Fragmente sind in Fig.10 dargestellt. Für die Globin Transkripte beider Expressions-Plasmide wird ein geschütztes Fragment von 360 Basen-Paaren erwartet. Wegen der Insertion von sieben Basen-Paaren in Exon I des Kaninchen ß-Globin Gens im Referenz-Plasmid wird ein wesentlich kürzeres geschütztes Fragment erwartet.

## Patentansprüche

### Patentansprüche für folgenden Vertragsstaaten: BE, CH, DE, FR, GB, IT, LI, LU, NL, SE

1. Verfahren zur gegenüber cDNA verbesserten Expression von Eukaryontenprotein, wobei man cDNA eines Eukaryontengens in Prokaryonten kloniert, die klonierte cDNA daraus gewinnt und dieselbe in eine Eukaryontenzelle einführt, welche die cDNA in das gewünschte Protein übersetzt,
**dadurch gekennzeichnet,**
daß man mindestens ein im entsprechenden Gen natürlich vorkommendes Intron gewinnt, in die kodierende Sequenz an geeigneter Stelle der cDNA einligiert und die so erhaltene Intron-haltige cDNA in Prokaryonten kloniert.

2. Verfahren nach Anspruch 1,
**dadurch gekennzeichnet,**
daß in die kodierende Sequenz der cDNA mehrere im entsprechenden Gen natürlich vorkommende Introns eingeführt werden.

3. Verfahren nach Anspruch 1 oder 2,
**dadurch gekennzeichnet,**
daß man das Intron an der Stelle in die cDNA einführt, welche der Stelle korrespondiert, die im entsprechenden Gen dieses Intron aufweist.

4. Verfahren nach Anspruch 1, 2 oder 3
**dadurch gekennzeichnet,**
daß die verwendete cDNA das Protein tPA codiert.

5. Verfahren nach Anspruch 4,
**dadurch gekennzeichnet,**
daß man das Intron C einführt.

6. Plasmid pBT 94 (DSM 3610).

7. Plasmid pBT 102 (DSM 3612).

8. Verfahren zur Herstellung des Plasmids pBT 94,
**dadurch gekennzeichnet,**
daß man in das Plasmid pBT 95 (DSM 3611), das die komplette tPA-cDNA einschließlich des Gens für das Signalpeptid enthält, das Intron C einligiert.

9. Verfahren zur Herstellung des Plasmids pBT 102,
**dadurch gekennzeichnet,**
daß man die aus dem Plasmid pBT 94 (DSM 3610) gewonnene vollständige Sequenz für tPA-cDNA einschließlich Intron in das Plasmid pA-11-SVL$_2$ (DSM 3614) einligiert.

### Patentansprüche für folgenden Vertragsstaaten: AT, ES, GR

1. Verfahren zur gegenüber cDNA verbesserten Expression von Eukaryontenprotein, wobei man cDNA eines Eukaryontengens in Prokaryonten kloniert, die klonierte cDNA daraus gewinnt und dieselbe in eine Eukaryontenzelle einführt, welche die cDNA in das gewünschte Protein übersetzt,
**dadurch gekennzeichnet,**
daß man mindestens ein im entsprechenden Gen natürlich vorkommendes Intron gewinnt, in die kodierende Sequenz an geeigneter Stelle der cDNA einligiert und die so erhaltene Intron-haltige cDNA in Prokaryonten kloniert.

2. Verfahren nach Anspruch 1,
**dadurch gekennzeichnet,**
daß in die kodierende Sequenz der cDNA mehrere im entsprechenden Gen natürlich vorkommende Introns eingeführt werden.

3. Verfahren nach Anspruch 1 oder 2,
**dadurch gekennzeichnet,**
daß man das Intron an der Stelle in die cDNA einführt, welche der Stelle korrespondiert, die im entsprechenden Gen dieses Intron aufweist.

4. Verfahren nach Anspruch 1, 2 oder 3
**dadurch gekennzeichnet,**
daß die verwendete cDNA das Protein tPA codiert.

5. Verfahren nach Anspruch 4,
**dadurch gekennzeichnet,**

daß man das Intron C einführt.

6. Verfahren zur Herstellung des Plasmids pBT 94,

**dadurch gekennzeichnet,**

daß man in das Plasmid pBT 95 (DSM 3611), das die komplette tPA-cDNA einschließlich des Gens für das Signalpeptid enthält, das Intron C einligiert.

7. Verfahren zur Herstellung des Plasmids pBT 102,

**dadurch gekennzeichnet,**

daß man die aus dem Plasmid pBT 94 (DSM 3610) gewonnene vollständige Sequenz für tPA-cDNA einschließlich Intron in das Plasmid pA-11-SVL$_2$ (DSM 3614) einligiert.


## Claims

### Claims for the following Contracting States: BE, CH, LI, DE, FR, GB, IT, LU, NL, SE

1. Process for the expression of eukaryote proteins improved with regard to cDNA, whereby one clones cDNA of a eukaryote gene into prokaryotes, recovers the cloned cDNA therefrom and introduces the same into a eukaryote cell which translates the cDNA into the desired protein,
characterised in that
one recovers at least one intron occurring naturally in the corresponding gene, ligates into the coding sequence at suitable position of the cDNA and clones the so-obtained intron-containing cDNA into prokaryotes.

2. Process according to claim 1,
characterised in that
into the coding sequence of the cDNA are introduced several introns occurring naturally in the corresponding gene.

3. Process according to claim 1 or 2,
characterised in that
one introduces the intron at the position in the cDNA which corresponds to the position which displays this intron in the corresponding gene.

4. Process according to claim 1, 2 or 3,
characterised in that
the cDNA used codes the protein tPA.

5. Process according to claim 4,
characterised in that
one introduces the intron C.

6. Plasmid pBT 94 (DSM 3610).

7. Plasmid cBT 102 (DSM 3612).

8. Process for the production of the plasmid pBT 94,
characterised in that
one ligates the intron C into the plasmid pBT 95 (DSM 3611) which contains the complete tPA-cDNA including the gene for the signal peptide.

9. Process for the production of the plasmid pBT 102,
characterised in that
one ligates the complete sequence for tPA-cDNA, obtained from the plasmid pBT 94 (DSM 3610), including intron, into the plasmid pA-11-SVL$_2$ (DSM 3614).

### Claims for the following Contracting States: AT, ES, GR

1. Process for the expression of eukaryote proteins improved with regard to cDNA, whereby one clones cDNA of an eukaryont gene into prokaryonts, recovers the cloned cDNA therefrom and introduces the same into a eukaryont cell which translates the cDNA into the desired protein,
characterised in that
one recovers at least one intron occurring naturally in the corresponding gene, ligates into the coding sequence at suitable position of the cDNA and clones the so-obtained intron-containing cDNA into prokaryotes.

2. Process according to claim 1,
characterised in that
several introns occurring naturally in the corresponding gene are introduced into the coding sequence of the cDNA.

3. Process according to claim 1 or 2,
characterised in that
one introduces the intron at the position in the cDNA which corresponds to the position which displays this intron in the corresponding gene.

4. Process according to claim 1, 2 or 3,
characterised in that
the cDNA used codes the protein tPA.

5. Process according to claim 4,
characterised in that
one introduces the intron C.

6. Process for the production of the plasmid pBT 94,
characterised in that
one ligates the intron C into the plasmid pBT 95 (DSM 3611) which contains the complete tPA-cDNA, including the gene for the signal peptide.

7. Process for the production of the plasmid pBT 102,
characterised in that
one ligates the complete sequence for tPA-cDNA, obtained from the plasmid pBT 94 (DSM 3610), including intron, into the plasmid pA-11-SVL$_2$ (DSM 3614).


**Revendications**

**Revendications pour les Etats contractants suivants: BE, CH, LI, DE, FR, GB, IT, LU, NL, SE**

1. Procédé d'expression améliorée, du point de vue de l'ADNc, d'une protéine eucaryotique, dans lequel on clone l'ADNc d'un gène eucaryotique dans des procaryotes, on isole de ceux-ci l'ADNc cloné et on l'introduit dans une cellule eucaryotique qui exprime l'ADNc en la protéine souhaitée,
caractérisé en ce que
l'on isole au moins un intron présent naturellement dans le gène correspondant, on l'introduit par ligature dans la séquence codante à l'endroit approprié de l'ADNc et on clone dans des procaryotes l'ADNc contenant l'intron ainsi obtenu.

2. Procédé selon la revendication 1,
caractérisé en ce que
l'on introduit dans la séquence codante de l'ADNc plusieurs introns présents naturellement dans le gène correspondant.

3. Procédé selon la revendication 1 ou 2,
caractérisé en ce que
l'on introduit l'intron à l'endroit de l'ADNc qui correspond à l'endroit qui présente cet intron dans le gène correspondant.

4. Procédé selon la revendication 1, 2 ou 3,
caractérisé en ce que
l'ADNc utilisé code pour la protéine tPA.

5. Procédé selon la revendication 4,
caractérisé en ce que
l'on introduit l'intron C.

6. Plasmide pBT 94 (DSM 3610).

7. Plasmide pBT 102 (DSM 3612).

8. Procédé de préparation du plasmide pBT 94,
caractérisé en ce que
l'on introduit par ligature l'intron C dans le plasmide pBT 95 (DSM 3611) qui contient l'ADNc complet du tPA y compris le gène du peptide signal.

9. Procédé de préparation du plasmide pBT 102,
caractérisé en ce que
l'on introduit par ligature dans le plasmide pA-11-SVL$_2$ (DSM 3614) la séquence complète, isolée à partir du plasmide pBT 94 (DSM 3610), de l'ADNc du tPA y compris l'intron.

**Revendications pour les Etats contractants suivants: AT, ES, GR**

1. Procédé d'expression améliorée, du point de vue de l'ADNc, d'une protéine eucaryotique, dans lequel on clone l'ADNc d'un gène eucaryotique dans des procaryotes, on isole de ceux-ci l'ADNc cloné et on l'introduit dans une cellule eucaryotique qui exprime l'ADNc en la protéine souhaitée,
caractérisé en ce que
l'on isole au moins un intron présent naturellement dans le gène correspondant, on l'introduit par ligature dans la séquence codante à l'endroit approprié de l'ADNc et on clone dans des procaryotes l'ADNc contenant l'intron ainsi obtenu.

2. Procédé selon la revendication 1,
caractérisé en ce que
l'on introduit dans la séquence codante de l'ADNc plusieurs introns présents naturellement dans le gène correspondant.

3. Procédé selon la revendication 1 ou 2,
caractérisé en ce que
l'on introduit l'intron à l'endroit de l'ADNc qui correspond à l'endroit qui présente cet intron dans le gène correspondant.

4. Procédé selon la revendication 1, 2 ou 3,
caractérisé en ce que
l'ADNc utilisé code pour la protéine tPA.

5. Procédé selon la revendication 4,
caractérisé en ce que
l'on introduit l'intron C.

6. Procédé de préparation du plasmide pBT 94,
caractérisé en ce que
l'on introduit par ligature l'intron C dans le plasmide pBT 95 (DSM 3611) qui contient l'ADNc complet du tPA y compris le gène du peptide signal.

7. Procédé de préparation du plasmide pBT 102,
caractérisé en ce que
l'on introduit par ligature dans le plasmide pA-11-SVL$_2$ (DSM 3614) la séquence complète, isolée à partir du plasmide pBT 94 (DSM 3610), de l'ADNc du tPA y compris l'intron.

FIG.1

Hind III — Pst I — Sal I

Polylinker

pHC 79

pBT 95
~5,2kb

Nar I

Polylinker
Pst I
Bgl II
Xba I
Xho II

FIG.2

Hind III — Pst I — Sal I

Polylinker

pHC 79

pBT 94
~7,4 kb

Nar I

Xho II
Xba I
Bgl II
Polylinker

Intron C

Pst I

# FIG.3

Pst I

Nar I

Intron C

Bgl II

BamHI

pBT 102
~11,9kb

SV40

Ori

Xba I

amp

pBR 322

14

# FIG.4

FIG.5

pKCR-tpA₁

SV 40 early
t-pA c DNS
JVS Kaninchen β-Globin
Exon III Kaninchen β-Globin
pA
SV 40
pA
pBR322
amp
Pvu I

FIG.6

pKCR-tpA₂

SV40 early
t-pA Minigen
JVS Kaninchen β-Globin
Exon III Kaninchen β-Globin
pA
SV 40
pA
pBR322
amp
Pvu I

# FIG .7

# FIG.8

(a)

(b)

(c)

# FIG . 9

Referenz – Plasmid RβG ref

# FIG . 10

β-globin cDNA

pβcg

Pst
-100

Pvu II
-10

Bam HI

450 bp — Probe

SV 40 early

SVβc

PvuII
-10

BamHI

360 bp geschützt

SV40 early

SVβg

PvuII
-10

BamH1

360 bp geschützt

Probe und geschützte Fragmente beim S1 mapping